# EUROPÄISCHE PATENTSCHRIFT

(11) **EP 3 800 496 B1**
(45) Veröffentlichungstag und Bekanntmachung des Hinweises auf die Patenterteilung: **26.10.2022**
(21) Anmeldenummer: 20196360.0
(22) Anmeldetag: 16.09.2020
(51) Int. Cl.: G02B 23/24

(54) **ENDOSKOPISCHES INSTRUMENT**
ENDOSCOPIC INSTRUMENT
INSTRUMENT ENDOSCOPIQUE

(30) Priorität: 01.10.2019 DE 202019105420 U
(43) Veröffentlichungstag der Anmeldung: 07.04.2021
(73) Patentinhaber: Richard Wolf GmbH, 75438 Knittlingen (DE)
(72) Erfinder: Schwab, Daniel, 75438 Knittlingen-Freudenstein (DE)
(74) Vertreter: Patentanwälte Vollmann Hemmer Lindfeld Partnerschaft mbB

(56) Entgegenhaltungen:
- WO-A1-2014/031955
- US-A1- 2016 081 708

## Beschreibung

Die Offenbarung betrifft ein technoskopisches Instrument zur Bearbeitung von Oberflächen in technischen Hohlräumen, wobei das Instrument einen feststehenden, rohrförmigen Schaft und eine distal an dem Schaft angeordnete Werkzeugeinheit zum Aufnehmen eines Bearbeitungswerkzeugs aufweist und relativ zu einer Längsachse des Schafts mittels eines Gelenks verschwenkbar ist, wobei die Werkzeugeinheit zum selektiven Verschwenken mit einer ersten Verstelleinheit gekoppelt ist, die proximal an dem Instrument angeordnet ist.

Ein derartiges technoskopisches Instrument kann zum Durchführen von Reparaturarbeiten an Komponenten in technischen Systemen genutzt werden, ohne dass eine aufwändige Demontage der betreffenden Komponente erfolgen muss. Die Werkzeugeinheit und der Schaft könnten hierfür durch eine geeignete Öffnung in das betreffende System eingeführt werden. Ein Benutzer kann während der Bedienung des Instruments ein von einer Optik erfasstes Bild betrachten, wobei diese unmittelbar an oder benachbart zu einem distalen Ende der Werkzeugeinheit mündet. Die Werkzeugeinheit kann verschiedenartige Bearbeitungswerkzeuge tragen, insbesondere rotierende Werkzeuge wie Bohrer, Fräser, Schleifer und dergleichen.

In derartigen technischen Hohlräumen können unterschiedlichste Anforderungen an die Bearbeitung durch das technoskopische Instrument gestellt werden, die möglicherweise unterschiedliche Werkzeuge erfordern. Je nach vorgefundenem Zustand der zu bearbeitenden Oberfläche könnten insbesondere einige Bereiche mit einem ersten Werkzeug besser erreicht werden als andere Bereiche, die ein anders geformtes zweites Werkzeug erfordern würden. Es ist jedoch mühsam und zeitintensiv, die Werkzeugeinheit nacheinander mit verschiedenen Werkzeugen auszustatten. Weiterhin erhöht das Bereitstellen mehrerer unterschiedlicher Bearbeitungswerkzeuge zur bedarfsweisen Verwendung die gebundenen Investitionen für das technoskopische Instrument. Die US 20016/081708 A1 beschreibt ein minimal-invasives medizinisches Endoskop mit abwinkelbarer Spitze. Die WO 2014/031955 A1 beschreibt ein optisches Kamerainspektionssystem für Gas- und Dampfturbinen.

Die Aufgabe der Erfindung liegt in einer verbesserten Nutzung eines einzelnen Werkzeugs im Vergleich zu bekannten technoskopischen Instrumenten dieser Art. Insbesondere soll die verbesserte Nutzung einen Arbeitsbereich der Werkzeugeinheit erweitern, ohne die Bedienbarkeit oder Flexibilität des Instruments einzuschränken.

Die Aufgabe wird durch ein technoskopisches Instrument mit den Merkmalen des Schutzanspruchs 1 gelöst. Vorteilhafte Weiterbildungen sind den Unteransprüchen sowie der nachfolgenden Beschreibung zu entnehmen.

Es wird ein technoskopisches Instrument zur Bearbeitung von Oberflächen in technischen Hohlräumen vorgeschlagen, wobei das Instrument einen feststehenden, rohrförmigen Schaft und eine distal an dem Schaft angeordnete Werkzeugeinheit zum Halten eines Bearbeitungswerkzeugs aufweist und quer zu einer Längsachse des Schafts mittels eines Gelenks verschwenkbar ist, wobei die Werkzeugeinheit zum selektiven Verschwenken mit einer ersten Verstelleinheit gekoppelt ist, die proximal an dem Instrument angeordnet ist. Erfindungsgemäß weist die schwenkbare Werkzeugeinheit einen distal zu dem Gelenk angeordneten Endabschnitt auf, der axial verschiebbar gelagert ist und über ein längliches Steuerelement, das zugstarr, schubsteif und quer zu seiner längsaxialen Erstreckung biegeweich ist, mit einer proximal an dem Instrument angeordneten zweiten Verstelleinheit, die von der ersten Verstelleinheit unabhängig und separat vorgesehen ist, zum selektiven Teleskopieren des Endabschnitts gekoppelt ist.

Bei dem erfindungsgemäßen technoskopischen Instrument ist folglich zusätzlich zu der Verschwenkbarkeit der Werkzeugeinheit eine unabhängig davon realisierte und einfache Teleskopierbarkeit vorgesehen. Folglich kann das an der Werkzeugeinheit angebrachte Bearbeitungswerkzeug in axialer Richtung relativ zu der Werkzeugeinheit bewegt werden, so dass sich diese bedarfsweise in axial verlängert oder verkürzt. Die von dem Bearbeitungswerkzeug durch gezielte Bedienung der beiden unabhängigen Verstelleinheiten erreichbare Fläche wird dadurch im Vergleich zu bekannten technoskopischen Instrumenten deutlich vergrößert. Ein aufwändiges Austauschen von Bearbeitungswerkzeugen ist für die überwiegenden Anwendungen daher nicht notwendig, um den Einsatzbereich zu vergrößern.

Der Endabschnitt der Werkzeugeinheit ist als ein proximaler, axialer Abschnitt der Werkzeugeinheit zu verstehen und wird durch eine axiale Unterteilung erreicht. Der Endabschnitt könnte beispielsweise axial an einem Schwenkabschnitt geführt werden, der mit dem Gelenk verbunden ist oder dieses aufweist. Der Endabschnitt kann dadurch in axialer Richtung relativ zu dem Schwenkabschnitt bewegt werden.

Die axiale Bewegung wird dabei durch das längliche Steuerelement erreicht, welches mit der zweiten Verstelleinheit gekoppelt ist. Die erste Verstelleinheit und die zweite Verstelleinheit sind separat voneinander bedienbar und können unabhängig voneinander das Verschwenken und Teleskopieren initiieren.

Das längliche Steuerelement verläuft von der zweiten Verstelleinheit zu dem Endabschnitt der Werkzeugeinheit und durchläuft damit das Gelenk. Aufgrund seiner biegeweichen Ausgestaltung kann das Steuerelement einer durch die Verschwenkung verursachten Richtungsänderung an dem Gelenk ohne weiteres folgen. Da das Steuerelement zudem zugstarr und schubsteif ist, führt eine auf das Steuerelement ausgeübte Kraft zu einer gezielten Bewegung des Endabschnitts. Es ist vorstellbar, dass das Steuerelement insbesondere als ein Draht oder ein drahtförmiges Element aus einem nicht-metallischen Material ausgeführt ist und sowohl als Zug- als auch als Schubmittel dient. Einerseits könnte es flexibel genug sein, um der verschwenkten Werkzeugeinheit zu folgen, doch andererseits ausreichend steif, um bei der Verwendung als Schubmittel nicht zu knicken.

Optional weist das Steuerelement mindestens einen Draht auf. Das Steuerelement könnte teilweise, weitgehend vollständig oder vollständig aus einem Draht oder mehreren Drähten bestehen. Je nach der zu überbrückenden Distanz zwischen dem Gelenk und einer vollständig ausgefahrenen Position des Endabschnitts ergibt sich ein geeigneter Durchmesser des Drahts, um gleichzeitig die erforderliche Schubsteifigkeit zu erhalten und dennoch ein Biegen des Steuerelements zum Folgen der Ausrichtung des Endabschnitts zu erlauben. Der Draht kann insbesondere ein rostfreier Stahldraht sein oder andere Materialien aufweisen. Der mindestens eine Draht kann auch als Litze mit mehreren, einander umschließenden Drähten ausgeführt sein. Des Weiteren ist denkbar, dass zumindest ein axialer Teilabschnitt durch eine Umhüllung geschützt ist, wobei die Umhüllung biegestarr sein könnte und in einem vorgesehenen Bereich des Instruments einer Verbiegung entgegenwirkt.

Weiter optional kann die Werkzeugeinheit einen Hülsenabschnitt und einen darin axial verschiebbaren Zylinderabschnitt aufweisen. Der Hülsenabschnitt ist ein Teil der Werkzeugeinheit und weist die Form einer Hülse auf. Der Hülsenabschnitt könnte an der zu dem Gelenk gewandten Seite der Werkzeugeinheit oder distal vorgesehen sein und damit den Endabschnitt realisieren. Der Zylinderabschnitt weist eine mit dem Hülsenabschnitt korrespondierende Form auf und kann in ein offenes Ende des Hülsenabschnitts gesteckt werden, um dort in axialer Richtung geführt zu werden. Befindet sich der Zylinderabschnitt distal an der Werkzeugeinheit, kann dieser den Endabschnitt darstellen.

Der Hülsenabschnitt könnte in einer vorteilhaften Ausführungsform mit dem Gelenk gekoppelt sein, wobei der Zylinderabschnitt als Endabschnitt mit dem Steuerelement verbunden ist. Er wird durch das Steuerelement axial verschoben und bestimmt dadurch die axiale Position des Bearbeitungswerkzeugs. Der Zylinderabschnitt kann an einem proximalen Ende ein Befestigungsmittel zum Befestigen des Steuerelements aufweisen. Das Befestigungsmittel könnte unter anderem eine Bohrung aufweisen, in die das Steuerelement geführt ist. Dort könnte es beispielsweise verklebt, verschweißt oder geklemmt sein.

Erfindungsgemäß ist das Steuerelement von einer Längsachse des Schafts beabstandet und nimmt in der Werkzeugeinheit eine koaxiale Ausrichtung mit der Werkzeugeinheit ein. Das Steuerelement verläuft folglich exzentrisch zu einer Längsachse des Schafts, insbesondere im Innern des Schafts und kann dadurch auf einfache Weise umfangsseitig an dem Gelenk vorbeigeführt werden. Da der Endabschnitt der Werkzeugeinheit axial zu verschieben ist, bietet sich eine mittige, konzentrische Position des Steuerelements an der Werkzeugeinheit an, um bei der axialen Bewegung ein Verkanten möglichst zuverlässig zu verhindern.

Die Werkzeugeinheit weist außerdem einen zu dem Gelenk gewandten Einlaufbereich für das Steuerelement auf, der sich in distaler Richtung verjüngt. In diesem Einlaufbereich läuft das Steuerelement aus proximaler Richtung in die Werkzeugeinheit hinein. Da durch das Verschwenken der Werkzeugeinheit stets ein korrespondierendes Biegen des Steuerelements erfolgt, kann das Steuerelement eine von einer Verschwenkung der Werkzeugeinheit abhängige Verlaufsrichtung relativ zu der Längserstreckung der Werkzeugeinheit einnehmen. Durch eine sich zu dem Gelenk hin erweiternde bzw. sich in distaler Richtung hin verjüngende Öffnung ergibt sich ein trichterförmiger Einlaufbereich, der eine Richtungsanpassung des Steuerelements nach Passieren des Gelenks erleichtert und die relative Ausrichtung toleriert.

Des Weiteren könnte der Schaft in einem zu dem Gelenk benachbarten Bereich mindestens ein von der Werkzeugeinheit beabstandetes Führungselement aufweisen, das eine Durchbiegung des Steuerelements in mindestens einer Richtung begrenzt. Das Führungselement erlaubt generell in einem bestimmten Schwenkbereich der Werkzeugeinheit ein ungestörtes Biegen des Steuerelements. Um jedoch einen Verlust der Schubkraftübertragung und/oder eine Beschädigung des Steuerelements zu verhindern, kann durch geeignete Platzierung des mindestens einen Führungselements eine Stärke der Durchbiegung begrenzt werden. Es ist weiterhin denkbar, zwei oder mehr Führungselemente vorzusehen, welche das Durchbiegen in zwei unterschiedlichen Richtungen begrenzen. Diese könnten etwa einander gegenüberliegen.

An dieser Stelle sei darauf hingewiesen, dass das Steuerelement zur weiteren Begrenzung seiner Durchbiegung durch einen in dem Instrument und insbesondere an dem Gelenk verlaufenden Spalt geführt sein könnte, der eine flächige Erstreckung mit Haupterstreckungsebenen quer zu einer Scharnierachse des Gelenks aufweist. Die Dicke des Spalts wäre dann etwas größer als das entsprechende Querschnittsmaß des Steuerelements zu wählen, so dass dessen ungehinderte Bewegung möglich ist.

Die Werkzeugeinheit kann optional zum Verschwenken exzentrisch mit einer exzentrisch in dem Schaft geführten Schubstange gekoppelt sein, die durch die erste Verstelleinheit bewegbar ist. Die Schubstange kann weiterhin ebenso durch ein teilweise flexibles oder biegeelastisches Element realisiert werden, welches beispielsweise zumindest teilweise mit einer Umhüllung versehen ist. Die Verwendung einer Schubstange erlaubt auch das Übertragen größerer Kräfte, um quer zu der Werkzeugeinheit auftretende Kräfte, insbesondere um bei der Bearbeitung an dem Bearbeitungswerkzeug entstehende Gegenkräfte aufnehmen zu können.

Es ist vorteilhaft, wenn das Gelenk oder der Schaft mindestens eine Anschlagfläche zum Begrenzen eines Schwenkwinkels der Werkzeugeinheit aufweist. Die Anschlagfläche ist dazu ausgebildet, in bestimmten Schwenkpositionen einen Flächenkontakt mit der Werkzeugeinheit einzunehmen. Dadurch wird eine mechanische Begrenzung des Schwenkwinkels erreicht, was einem Benutzer die Bedienung des Instruments dadurch erleichtert, dass beim Bedienen der ersten Verstelleinheit eine klare mechanische Rückmeldung über die erreichte Endposition erfolgt. Weiterhin wird verhindert, dass ein übermäßiges Verschwenken und ein unbeabsichtigter Kontakt der Bearbeitungsfläche mit Oberflächen des technischen Hohlraums erfolgen. Durch Verhindern eines übermäßigen Biegens des Steuerelements kann so einem ungewünschten Knicken vorgebeugt werden.

Besonders vorteilhaft ist die Werkzeugeinheit um bis zu 120° verschwenkbar. Dieser Schwenkbereich ist ausreichend groß, um eine deutliche Vergrößerung des Arbeitsbereichs zu ermöglichen. Gleichzeitig kann das Steuerelement der Werkzeugeinheit über einen solchen Schwenkbereich gut folgen, ohne dass eine Gefahr eines Knickens entsteht. Sollte das Steuerelement in einem Spalt angeordnet sein, könnte dieser weiterhin derart dimensioniert sein, dass ein harmonischer, belastungsgerechter Verlauf des Steuerelements in dem Spalt möglich ist und ein Verspannen oder Reiben des Steuerelements verhindert wird.

Besonders bevorzugt ist der Endabschnitt drehfest verschiebbar. Eine Übertragung von Drehmoment zum Antreiben eines Bearbeitungswerkzeugs und die Aufnahme von Gegenkräften bei dem Betrieb eines Bearbeitungswerkzeugs mit verschwenkter Werkzeugeinheit ist dadurch ohne weitere Maßnahmen möglich.

Nachfolgend ist die Erfindung anhand von in den Zeichnungen dargestellten Ausführungsbeispielen näher erläutert. Es zeigen:
- Fig. 1: eine Seitenansicht eines technoskopischen Instruments;
- Fig. 2a, b: zwei Seitenansichten mit verschwenkter und nicht verschwenkter Werkzeugeinheit;
- Fig. 3a-c: drei Seitenansichten mit teleskopiertem und nicht teleskopiertem Endabschnitt, wobei Fig. 3b und 3c teilgeschnitten sind; und
- Fig. 4: eine Detailschnittdarstellung eines Gelenks.

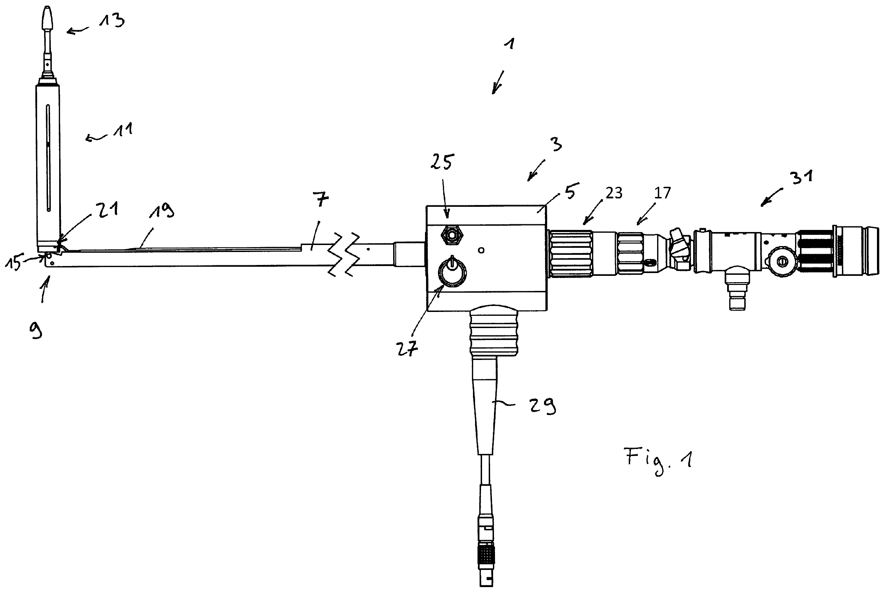

Fig. 1 zeigt eine Seitenansicht eines technoskopischen Instruments 1 zur Bearbeitung von Oberflächen in technischen Hohlräumen. Das Instrument 1 weist einen Handgriff 3 mit einem Gehäuse 5 auf, an dem ein feststehender, rohrförmiger Schaft 7 angeordnet ist. An einem distalen Ende des Schafts 7 ist ein Gelenk 9 angeordnet, das eine Werkzeugeinheit 11 trägt. Diese stellt eine distale Verlängerung des Schafts 7 dar und ist dazu ausgebildet, Bearbeitungswerkzeuge 13 zu halten. Das Gelenk 9 weist zum Erreichen einer Schwenkbarkeit zumindest eine Scharnierachse 15 auf, welche quer zu dem Schaft 7 verläuft.

Eine erste Verstelleinheit 17 ist an einem dem Schaft 7 entgegengesetzten Ende des Gehäuses 5 angeordnet und beispielhaft als ein Verstellrad mit einer umfangsseitigen Rändelung ausgeführt. Die erste Verstelleinheit 17 ist exemplarisch mit einer Schubstange 19 verbunden, welche exzentrisch in dem Schaft 7 in Richtung des Gelenks 9 verläuft. Dort ist sie mit der Werkzeugeinheit 11 an einem dem Gelenk 9 nahen, exzentrisch angeordneten Schwenkpunkt 21 gekoppelt. Die Schubstange 19 kann verschiebbar gelagert oder lediglich frei in dem Schaft 7 verlaufend angeordnet sein. Die erste Verstelleinheit 17 ist durch einen hier nicht dargestellten Mechanismus ausgebildet, die Schubstange 19 derart entlang des Schafts 7 zu bewegen, dass die Werkzeugeinheit 11 um die Scharnierachse 15 herumgeschwenkt wird.

Eine an die erste Verstelleinheit 17 distalseitig angrenzend angeordnete zweite Verstelleinheit 23 ist ebenfalls exemplarisch als Verstellrad mit umfangsseitiger Rändelung ausgeführt. In der gezeigten Variante sind die erste Verstelleinheit 17 und die zweite Verstelleinheit 23 koaxial zueinander ausgeführt und axial hintereinander angeordnet. Die zweite Verstelleinheit 23 ist, wie weiter nachfolgend ausgeführt, dazu vorgesehen, eine axiale Verschiebung des Bearbeitungswerkzeugs 13 zu erreichen.

An einer Außenseite des Gehäuses 5 befindet sich beispielhaft ein Schalter 25 zum bedarfsweisen Ein- oder Ausschalten eines Betriebs des Bearbeitungswerkzeugs 13. Dieses könnte rotierend ausgeführt sein. Eine Drehzahl des Bearbeitungswerkzeugs 13 könnte beispielhaft über einen ebenfalls an dem Gehäuse 5 angeordneten Drehzahlregler 27 gesteuert werden. An einem in der Zeichnungsebene unteren Ende schließt sich eine Versorgungsleitung 29 an, die beispielsweise der Stromversorgung eines Antriebs des Bearbeitungswerkzeugs 13 dient. Es ist denkbar, dass das Bearbeitungswerkzeug 13 mit einem Elektromotor ausgestattet ist, der in den Darstellungen der Einfachheit halber nicht im Detail gezeigt ist und entweder direkt an dem distalen Ende der Werkzeugeinheit 11 oder innerhalb des Gehäuses 5 platziert sein könnte. Schließlich befindet sich ein starres Endoskop 31 an einem proximalen Ende des Instruments 1 und erlaubt mithilfe einer geeigneten Optik eine Betrachtung des Arbeitsbereichs durch einen Benutzer. Die Optik könnte hierbei bis in die Werkzeugeinheit verlaufen.

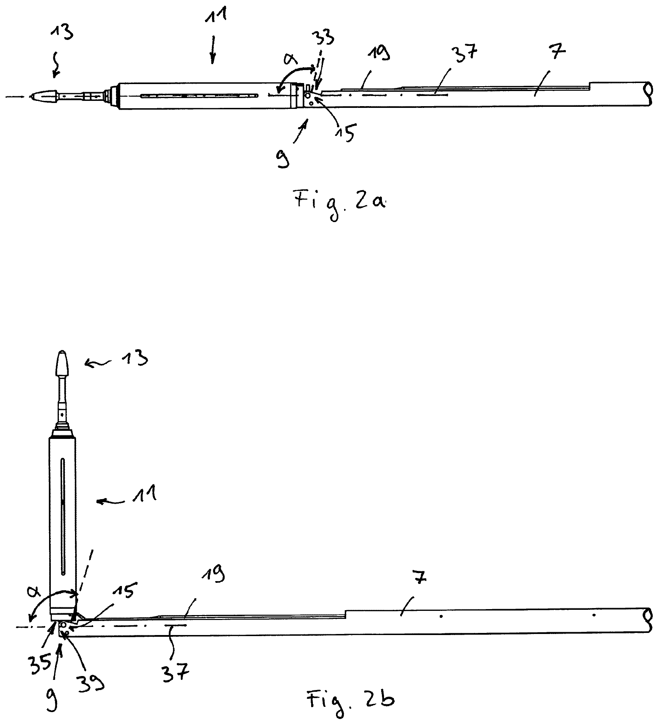

In den Figuren 2a und 2b wird das Verschwenken der Werkzeugeinheit 11 in zwei Seitenansichten dargestellt. Fig. 2a illustriert die Werkzeugeinheit 11 in einer nicht verschwenkten Position, in der der feststehende Schaft 7 und die Werkzeugeinheit 11 konzentrisch zueinander angeordnet sind. Die Schubstange 19 befindet sich in einer maximal ausgefahrenen distalen Position und der Schwenkpunkt 21 befindet sich in seiner distalen Endlage. In Fig. 2b ist die Werkzeugeinheit 11 in einer annähernd maximal verschwenkten Position gezeigt.

Der Schaft 7 weist in der Nähe des Gelenks 9 eine erste Anschlagfläche 33 auf, welche lateral an dem Schaft 7 angeordnet und dazu ausgebildet ist, mit einer proximalen Endfläche 35 der Werkzeugeinheit 11 in einen Flächenkontakt zu geraten. Dadurch wird ein möglicher Schwenkbereich der Werkzeugeinheit 11 auf einen durch die erste Anschlagfläche 33 vorgegebenen Winkelbereich a begrenzt. Dieser könnte beispielsweise einen Bereich von bis zu 120° umfassen und liegt zwischen der Flächennormalen auf der ersten Anschlagfläche 33 und einer Längsachse 37 des Schafts 7. Die in Fig. 2a gezeigte geradlinige, konzentrische Ausrichtung der Werkzeugeinheit 11 zu dem Schaft 7 korrespondiert ferner mit einer zweiten Anschlagfläche 39, welche hier senkrecht zu der Längsachse 37 ausgerichtet ist.

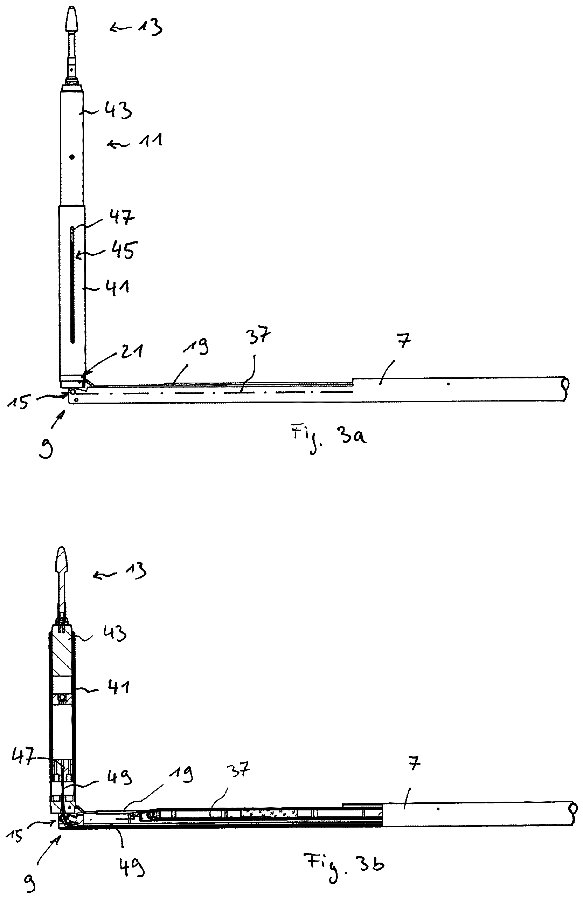

Wie in den Figuren 3a bis 3c dargestellt, kann die Werkzeugeinheit 11 axial teleskopiert werden, so dass das Bearbeitungswerkzeug 13 axial verschoben wird. In Fig. 3a befindet sich die Werkzeugeinheit 11 in einer ausgefahrenen Position. Hierfür weist die Werkzeugeinheit 11 einen an dem Gelenk 9 gelagerten Hülsenabschnitt 41 und einen darin axial verschiebbaren Zylinderabschnitt 43 als Endabschnitt auf, der die distale Erstreckung der Werkzeugeinheit 11 bestimmt. Der Zylinderabschnitt 43 weist einen Außendurchmesser auf, der geringfügig kleiner ist, als der Innendurchmesser des ihn umgebenden Hülsenabschnitts 41. Hierdurch ergibt sich eine einfache und dennoch robuste mechanische Führung. Um ein Verdrehen des Zylinderabschnitts 43 zu verhindern, weist der Hülsenabschnitt 41 zudem mindestens einen axial verlaufenden Schlitz 45 auf, in dem ein Querstift 47 des Zylinderabschnitts 43 geführt ist. Es ist denkbar, dass der Hülsenabschnitt 41 auf zwei einander gegenüberliegenden Seiten einen solchen Schlitz 45 besitzt, durch den sich ein einzelner oder mehrere Querstifte 47 erstrecken. Durch das Verschieben des Zylinderabschnitts 43 wird der Aktionsradius des Bearbeitungswerkzeugs 13 deutlich vergrößert.

Zum Bewegen des Zylinderabschnitts 43 ist dieser mit einem länglichen Steuerelement 49 gekoppelt, das exzentrisch in dem feststehenden Schaft 7 geführt und durch die erste Verstelleinheit 17 längsaxial bewegbar ist. Das Steuerelement 49 ist quer zu seiner Längsachse biegeweich, jedoch zugstarr und schubsteif. Es kann zumindest abschnittsweise ähnlich wie ein Bowdenzug ausgeführt sein. Das Steuerelement 49 ist durch den biegeweichen Charakter in der Lage, über den gesamten Schwenkbereich an dem Gelenk 9 vorbeigeführt zu werden. Wird die Werkzeugeinheit 11 verschwenkt, kann das Steuerelement 49 dieser Bewegung sehr leicht folgen. Wird es längsaxial bewegt, kann der Zylinderabschnitt 43 dieser Bewegung folgen. Aufgrund der zug- und schubstarren Eigenschaften kann der Zylinderabschnitt 43 sowohl aus- als auch eingefahren werden.

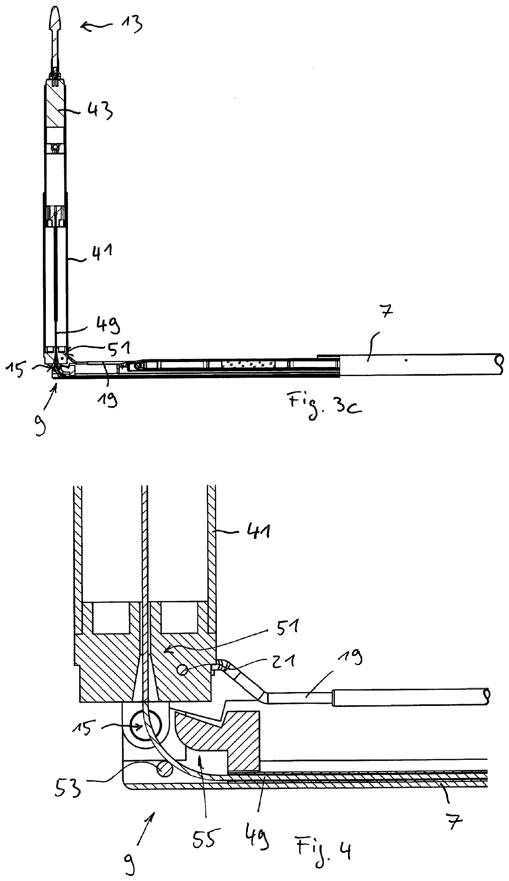

Fig. 3c zeigt beispielsweise die ausgefahrene Position der Werkzeugeinheit 11, bei der das Steuerelement 49 vollständig ausgeschoben ist. Aufgrund der biegeweichen Eigenschaften ist das Steuerelement 49 jedoch nicht über eine unbegrenzte Strecke in der Lage, Schubkräfte zu übertragen, ohne dass seine Bewegbarkeit in Querrichtung eingeschränkt wird. Hierfür sind in der gezeigten Darstellung Führungselemente eingesetzt, welche anhand Fig. 4 deutlich werden und eine Durchbiegung des Steuerelements 49 begrenzen.

Fig. 4 zeigt in einer geschnittenen Detailansicht den Verlauf des Steuerelements 49 im Bereich des Gelenks 9. Der Hülsenabschnitt 41 weist zum Führen des Steuerelements 49 einen Einlaufbereich 51 auf, der sich in distaler Richtung verjüngt. Hierdurch ist der zu dem Gelenk 9 gewandte Bereich des Einlaufbereichs 51 in etwa trichterförmig ausgestaltet. Exemplarisch ist der Einlaufbereich 51 konzentrisch in der Werkzeugeinheit 11 angeordnet und führt das Steuerelement 49 mittig an den Zylinderabschnitt heran, so dass bei dessen Bewegung die Gefahr eines Verkantens minimiert wird. Durch den Einlaufbereich 51 kann das Steuerelement 49 auch unterschiedlich verschwenkten Werkzeugeinheiten 11 folgen, ohne dass es an einer scharfkantigen Öffnung entlangreibt.

Zum Führen des Steuerelements 49 zum Begrenzen einer Durchbiegung kann der Schaft 7 exemplarisch ein erstes Führungselement 53 aufweisen, das in einem zu dem Gelenk 9 benachbarten Bereich positioniert und von der Werkzeugeinheit 11 beabstandet ist. In einem einfachen Fall kann das erste Führungselement 53 als ein Stift ausgeführt sein, der quer zu der Längsachse 37 des Schafts 7 verläuft und in zumindest einem Winkelbereich der Werkzeugeinheit 11 in einen Flächenkontakt mit dem Steuerelement 49 geraten kann. Dann wird eine darüberhinausgehende Durchbiegung zuverlässig verhindert.

Des Weiteren könnte ein quer zu der längsaxialen Erstreckung des Steuerelements 49 von dem ersten Führungselement 53 beabstandetes und proximal etwas versetztes zweites Führungselement 55 vorgesehen sein. Beispielhaft weist das zweite Führungselement 55 eine konvexe Oberfläche auf, die ebenso in einen Flächenkontakt mit dem Steuerelement 49 geraten kann. Das Steuerelement 49 befindet sich dann stets zwischen den beiden Führungselementen 53 und 55 und ist dann zumindest von einem der beiden Führungselemente 53 und 55 beabstandet. Wird die Werkzeugeinheit 11 in eine koaxiale Lage mit dem Schaft 7 gebracht, schmiegt sich das Steuerelement 49 an das zweite Führungselement 55 an, so dass dessen Durchbiegung begrenzt wird und das Steuerelement 49 weiter Zug- und Schubkräfte übertragen kann. Bei einem hohen Schwenkwinkel gerät das Steuerelement 49 wiederum mit dem ersten Führungselement 53 in Flächenkontakt.

In Fig. 4 wird auch der Schwenkpunkt 21 näher gezeigt, der mit der Schubstange 19 gekoppelt ist. Die Schubstange 19 könnte über eine Gelenkgabel mit dem Schwenkpunkt 21 gekoppelt sein oder lediglich ein abgewinkeltes Ende aufweisen, das in die Werkzeugeinheit 11 gesteckt ist. Die gelenkige Verbindung führt zu einer Kippbewegung der Werkzeugeinheit 11 um das Gelenk 9 bzw. die dadurch bestimmte Scharnierachse 15.

### Bezugszeichenliste:

- 1: Technoskopisches Instrument
- 3: Handgriff
- 5: Gehäuse
- 7: feststehender Schaft
- 9: Gelenk
- 11: Werkzeugeinheit
- 13: Bearbeitungswerkzeug
- 15: Scharnierachse
- 17: erste Verstelleinheit
- 19: Schubstange
- 21: Schwenkpunkt
- 23: zweite Verstelleinheit
- 25: Schalter
- 27: Drehzahlregler
- 29: Versorgungsleitung
- 31: Endoskop
- 33: erste Anschlagfläche
- 35: proximale Endfläche
- 37: Längsachse
- 39: zweite Anschlagfläche
- 41: Hülsenabschnitt
- 43: Zylinderabschnitt, Endabschnitt
- 45: Schlitz
- 47: Querstift
- 49: Steuerelement
- 51: Einlaufbereich
- 53: erstes Führungselement
- 55: zweites Führungselement

## Patentansprüche

1. Technoskopisches Instrument (1) zur Bearbeitung von Oberflächen in technischen Hohlräumen, wobei das Instrument (1) einen feststehenden, rohrförmigen Schaft (7) und eine distal an dem Schaft (7) angeordnete Werkzeugeinheit (11) zum Halten eines Bearbeitungswerkzeugs (13) aufweist und quer zu einer Längsachse (37) des Schafts (7) mittels eines Gelenks (9) verschwenkbar ist, wobei die Werkzeugeinheit (11) zum selektiven Verschwenken mit einer ersten Verstelleinheit (17) gekoppelt ist, die proximal an dem Instrument (1) angeordnet ist,
wobei
die schwenkbare Werkzeugeinheit (11) einen distal zu dem Gelenk (9) angeordneten Endabschnitt (43) aufweist, der axial verschiebbar gelagert ist und über ein längliches Steuerelement (49), das zugstarr, schubsteif und quer zu seiner längsaxialen Erstreckung biegeweich ist, mit einer proximal an dem Instrument (1) angeordneten zweiten Verstelleinheit (23), die von der ersten Verstelleinheit (17) unabhängig und separat vorgesehen ist, zum selektiven Teleskopieren des Endabschnitts (43) gekoppelt ist,
**dadurch gekennzeichnet, dass** das Steuerelement (49) von einer Längsachse (37) des Schafts (7) beabstandet ist und in der Werkzeugeinheit (11) eine koaxiale Ausrichtung mit der Werkzeugeinheit (11) einnimmt, wobei die Werkzeugeinheit (11) einen zu dem Gelenk (9) gewandten Einlaufbereich (51) für das Steuerelement (49) aufweist, der sich in distaler Richtung verjüngt.

2. Technoskopisches Instrument (1) nach Anspruch 1, **dadurch gekennzeichnet, dass** das Steuerelement (49) mindestens einen Draht aufweist.

3. Technoskopisches Instrument (1) nach Anspruch 1 oder 2, **dadurch gekennzeichnet, dass** die Werkzeugeinheit (11) einen Hülsenabschnitt (41) und einen darin axial verschiebbaren Zylinderabschnitt (43) aufweist.

4. Technoskopisches Instrument (1) nach Anspruch 3, **dadurch gekennzeichnet, dass** der Hülsenabschnitt (41) mit dem Gelenk (9) gekoppelt ist und der Zylinderabschnitt (43) als Endabschnitt (43) mit dem Steuerelement (49) verbunden ist.

5. Technoskopisches Instrument (1) nach einem der vorhergehenden Ansprüche, **dadurch gekennzeichnet, dass** der Schaft (7) in einem zu dem Gelenk (9) benachbarten Bereich mindestens ein von der Werkzeugeinheit (11) beabstandetes Führungselement (53, 55) aufweist, das eine Durchbiegung des Steuerelements (49) in mindestens einer Richtung begrenzt.

6. Technoskopisches Instrument (1) nach einem der vorhergehenden Ansprüche, **dadurch gekennzeichnet, dass** die Werkzeugeinheit (11) zum Verschwenken exzentrisch mit einer exzentrisch in dem Schaft geführten Schubstange (19) gekoppelt ist, die durch die erste Verstelleinheit (17) bewegbar ist.

7. Technoskopisches Instrument (1) nach einem der vorhergehenden Ansprüche, **dadurch gekennzeichnet, dass** das Gelenk (9) oder der Schaft (7) mindestens eine Anschlagfläche (33, 39) zum Begrenzen eines Schwenkwinkels (a) der Werkzeugeinheit (11) aufweist.

8. Technoskopisches Instrument (1) nach einem der vorhergehenden Ansprüche, **dadurch gekennzeichnet, dass** die Werkzeugeinheit (11) um bis zu 120° verschwenkbar ist.

9. Technoskopisches Instrument (1) nach einem der vorhergehenden Ansprüche, **dadurch gekennzeichnet, dass** der Endabschnitt (43) drehfest verschiebbar ist.

## Claims

1. Technoscopic instrument (1) for machining surfaces in technical hollow spaces, wherein the instrument (1) comprises a stationary tubular shaft (7) and a tool unit (11), which is arranged distally on the shaft (7), for holding a machining tool (13) and which can be swivelled perpendicularly to a longitudinal axis (37) of the shaft (7) by means of joint (9), wherein for selective swivelling, the tool unit (11) is coupled to a first adjustment unit (17) that is arranged proximally on the instrument (1),
wherein the swivellable tool unit (11) comprises an end section (43) which is arranged distally with regard to the joint (9), is borne in an axially displaceable manner and via an elongated control element (49) that does not yield to tension, is rigid and bends perpendicularly to its longitudinal axial extension, is coupled a second adjustment unit (23) arranged proximally on the instrument (1), which is provided independently of and separately from the first adjustment unit (17), for the selective telescoping of the end section (43),
**characterised in that** the control element (49) is at a distance from a longitudinal axis (37) of the shaft (7) and in the tool unit (11) assumes a coaxial alignment with the tool unit (11), wherein, facing the joint (9), the tool unit (11) has an inlet area (51) for the control elements (49) that tapers in the distal direction.

2. Technoscopic instrument (1) according to claim 1, **characterised in that** the control element (49) comprises at least one wire.

3. Technoscopic instrument (1) according to claim 1 or 2, **characterised in that** the tool unit (11) comprises a sleeve section (41) and a cylinder section (43) that is axially displaceable therein.

4. Technoscopic instrument (1) according to claim 3, **characterised in that** the sleeve section (41) is coupled to the joint (9) and the cylinder section (43) is connected as an end section (43) to the control element (49).

5. Technoscopic instrument (1) according to any one of the preceding claims, **characterised in that** in an area adjacent to the joint (9), the shaft (7) comprises at least one guide element (53, 55) at a distance from the tool unit (11), which limits deflection of the control element (49) in at least one direction.

6. Technoscopic instrument (1) according to at least one of the preceding claims, **characterised in that** for swivelling, the tool unit (11) is eccentrically coupled to a push rod (19) which is eccentrically guided in the shaft and can be moved by the first adjustment unit (17).

7. Technoscopic instrument (1) according to any one of the preceding claims, **characterised in that** the joint (9) or the shaft (7) comprises at least one stop surface (33, 39) for limiting a swivel angle (a) of the tool unit (11).

8. Technoscopic instrument (1) according to any one of the preceding claims, **characterised in that** the tool unit (11) can be swivelled by up to 120°.

9. Technoscopic instrument (1) according to any one of the preceding claims, **characterised in that** the end section (43) is displaceable in a non-rotatable manner.

## Revendications

1. Instrument endoscopique (1) destiné au traitement de surfaces dans des cavités techniques, dans lequel l'instrument (1) comporte une tige tubulaire fixe (7) et une unité d'outil (11) agencée de manière distale sur la tige (7) destinée au maintien d'un outil de traitement (13), et qui peut pivoter transversalement à un axe longitudinal (37) de la tige (7) au moyen d'une articulation (9), l'unité d'outil (11) étant couplée, pour le pivotement sélectif, à une première unité de réglage (17) agencée de manière proximale sur l'instrument (1),
dans lequel l'unité d'outil pivotante (11) comporte une partie d'extrémité (43), agencée de manière distale par rapport à l'articulation (9), qui est montée de façon à pouvoir coulisser dans la direction axiale et qui, par l'intermédiaire d'un élément de commande oblong (49) qui est rigide en traction, rigide en poussée et souple en flexion transversalement à son étendue axiale longitudinale, est couplée à une seconde unité de réglage (23), agencée de manière proximale sur l'instrument (1), qui est prévue séparément et indépendamment de la première unité de réglage (17) et destinée au télescopage de la partie d'extrémité (43),
**caractérisé en ce que** l'élément de commande (49) est espacé d'un axe longitudinal (37) de la tige (7) et, dans l'unité d'outil (11), est en alignement coaxial avec l'unité d'outil (11), l'unité d'outil (11) comportant une zone d'entrée (51) de l'élément de commande (49) orientée vers l'articulation (9) et qui s'effile en direction distale.

2. Instrument endoscopique (1) selon la revendication 1, **caractérisé en ce que** l'élément de commande (49) comporte au moins un fil.

3. Instrument endoscopique (1) selon la revendication 1 ou 2, **caractérisé en ce que** l'unité d'outil (11) comporte une partie formant manchon (41) et une partie cylindrique (43) pouvant coulisser à l'intérieur de celle-ci dans la direction axiale.

4. Instrument endoscopique (1) selon la revendication 3, **caractérisé en ce que** la partie formant manchon (41) est couplée à l'articulation (9) et la partie cylindrique (43) est raccordée en tant que partie d'extrémité (43) à l'élément de commande (49).

5. Instrument endoscopique (1) selon l'une des revendications précédentes, **caractérisé en ce que** la tige (7) comporte, dans une région adjacente à l'articulation (9), au moins un élément de guidage (53, 55) espacé de l'unité d'outil (11), qui limite une flexion de l'élément de commande (49) dans au moins une direction.

6. Instrument endoscopique (1) selon l'une des revendications précédentes, **caractérisé en ce que** l'unité d'outil (11), pour le pivotement, est couplée de manière excentrée à une tige de poussée (19) guidée excentriquement dans la tige, qui est déplaçable par le biais de la première unité de réglage (17).

7. Instrument endoscopique (1) selon l'une des revendications précédentes, **caractérisé en ce que** l'articulation (9) ou la tige (7) comporte au moins une surface de butée (33, 39) destinée à limiter un angle de basculement (a) de l'unité d'outil (11).

8. Instrument endoscopique (1) selon l'une des revendications précédentes, **caractérisé en ce que** l'unité d'outil (11) peut pivoter jusqu'à 120°.

9. Instrument endoscopique (1) selon l'une des revendications précédentes, **caractérisé en ce que** la partie d'extrémité (43) peut coulisser de manière solidaire en rotation.
